# EUROPEAN PATENT SPECIFICATION

(11) **EP 2 438 883 B1**
(45) Date of publication and mention of the grant of the patent: **03.07.2013**
(21) Application number: 11184235.7
(22) Date of filing: 06.10.2011
(51) Int. Cl.: A61B 19/00, A47C 7/14, A47C 9/00, A47C 7/02

(54) **Stool, in particular for dentistry**
Hocker, insbesondere für die Zahnmedizin
Tabouret, en particulier pour la dentisterie

(30) Priority: 08.10.2010 IT BO20100599
(43) Date of publication of application: 11.04.2012
(73) Proprietor: CEFLA SOCIETA' COOPERATIVA, 40026 Imola (BO) (IT)
(72) Inventor: Nussbaumer, Christoph, 42017 Novellara (IT)
(74) Representative: Bergadano, Mirko

(56) References cited:
- WO-A1-93/17604
- NL-A- 6 905 431
- NL-A- 9 001 319
- US-A- 3 446 532
- US-A- 5 536 067
- US-A1- 2003 080 595

## Description

The present invention relates to a stool.

The present invention finds a particularly advantageous application in dentist's stools, to which explicit reference will be made in the present description without for this loosing in generality.

Since '60s, a sitting working position has become more and more common, while up to that moment dentists used to work standing. This pushed manufacturing companies to provide, in addition to the dental workstation, a stool able to support the dentist during dental therapies.

Therefore, in addition to dental units and patient chairs, manufacturers have been producing stools for accommodating the dentist during the performance of dental therapies.

US3726560 patent relates to a stool mounted on wheels wherein both the seat can be adjusted in height relative to the ground, and the angle of back rest and seat can be adjusted relative to the ground.

The European Society of Dental Ergonomics (ESDE) pointed out that dentistry is a very stressful profession, leading to musculo-skeletal complaints of different nature: 2/3 of dentists are supposed to be affected in different ways. A correct working posture becomes therefore very important, because an incorrect working posture can favour the onset of musculo-skeletal disorders.

In 2006 ESDE published a guideline (http://www.esde.org/docs/ergonomic_requirements_for_dental equipment._april2007.pdf) containing recommendations for designing dental equipment. This guideline also comprises indications concerning the dimensions and the adjustments that a dentistry stool should have. In particular, Chapter 10 of the document, titled Working stool dentist, states that the seat should be divided into two portions: a rear portion parallel to the ground, and a front portion which can be tilted toward the ground up to 20°, in order to allow the dentist to have an angle of about 110° between upper and lower leg.

A stool complying with ESDE requirements is described in application WO2005030007, wherein the rear section is fixed, while the tiltable front section is hinged relative to the rear section; moreover, the front section is linked to a vertical support post through the interposition of a crank.

Application WO2009030732 can be considered an evolution of the preceding application, wherein the front section provides at least two panels, each defining a respective rest plane for upper legs, while the panels are interconnected to each other so as to oscillate one up and the other down according to respective, substantially identical, angles.

Document NL9001319, which forms the basis for the preamble of claim 1, relates to a stool provided with a seat comprising a fixed rear portion and a tiltable front portion hinged to the rear portion itself. The front portion is mobile between a lifted position and a lowered position under the thrust of a crank gear comprising a crankshaft mounted so as to turn around a horizontal rotation axis, and a connecting rod interposed between the crankshaft and the anterior portion itself. The shifting of the front portion is operated by the operator, who has to grasp the crankshaft with his/her hand and rotate it around the said horizontal rotation axis.

It is known that in dentistry the so-called "universal precautions" must be used, because both blood and saliva can transmit dangerous pathogens (hepatitis B and C virus, *Mycobacterium tuberculosis).* Dental personnel normally work wearing gloves, and dental manufacturers do their utmost to limit the use of hands in imparting commands to dental equipment. By way of example, many commands can be given to the dental workstation through a foot pedal; there are washbasins wherein the water can be started through elbow or a photoelectric cell.

Aim of the present invention is providing a stool, in particular for dentistry, free from the above mentioned drawbacks, and that is of simple and economic construction, allowing the dentist to adjust the position of seat front portion without contaminating stool surfaces with his/her hands.

According to the present invention there is provided a stool, in particular for dentistry, according to the attached claims.

A non-limiting embodiment of the present invention will be now described by way of example with reference to the accompanying drawings, in which:
Figure 1 and 2 are two schematic lateral views of a preferred embodiment of the stool of the present invention, shown in two different operating positions:
Figure 3 is a perspective bottom view of the stool shown in Figures 1 and 2;
Figure 4 is a perspective bottom view of a detail of Figure 3; and
Figure 5 is an exploded schematic view of the detail of Figure 4; and
Figure 6 is a perspective view of a detail of Figure 5.

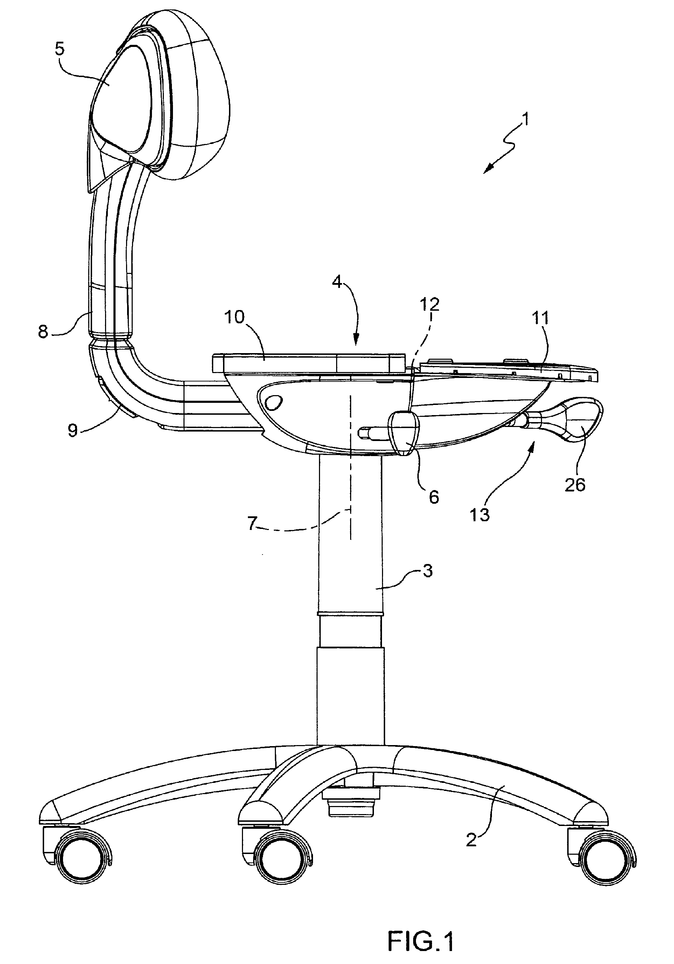

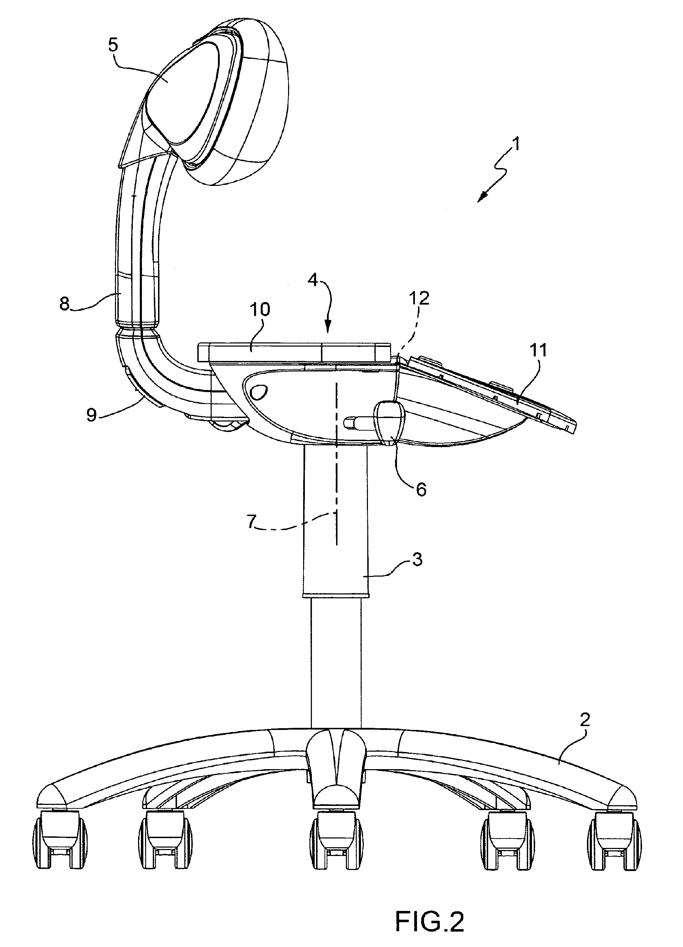

With reference to Figures 1 and 2, number 1 indicates as a whole a dental stool comprising a wheeled base 2, a telescopic post 3 protruding upwards from base 2, a seat 4 mounted on the top free end of post 3, and a backrest 5.

Moreover, stool 1 is provided with a lever 6, allowing to adjust the height of seat 4 relative to ground. Lever 6 is mounted to rotate around a substantially vertical rotation axis 7 and operates a known, not illustrated, gas spring.

Following the operation of lever 6, the gas spring is unblocked and thrusts seat 4 upwards with a force always contrary to that exerted by the dentist. As a consequence, combining the operation of lever 6 with the weight of the dentist seated on stool 1, seat 4 is moved downwards.

The rotation of lever 6 around axis 7, i.e. around a vertical rotation axis, allows the dentist to operate lever 6 with the back of his/her hand only, and prevents, therefore, that blood and/or saliva traces, that might be present on dentist's hands, contaminate lever 6 surface itself.

Moreover, it should be pointed out that backrest 5 is vertically adjustable relative to seat 4, and that the assembly including back rest 5 and a support 8 of backrest 5 is horizontally mobile relative to seat 4 between a backward position (Figure 1) and a forward position (Figure 2) under the thrust of a lever 9 mounted on the bottom part of support 8.

Seat 4 comprises a rear, substantially horizontal, portion 10 fixed to post 3 and a mobile front portion 11 hinged to portion 10 to rotate, relative to portion 10 itself, around a substantially horizontal rotation axis 12 between a lifted position (Figure 1), wherein portions 10 and 11 are substantially coplanar to each other, and a lowered position (Figure 2) wherein portions 10 and 11 are arranged according to an angle comprised, in the case in point, between 0° and 20°.

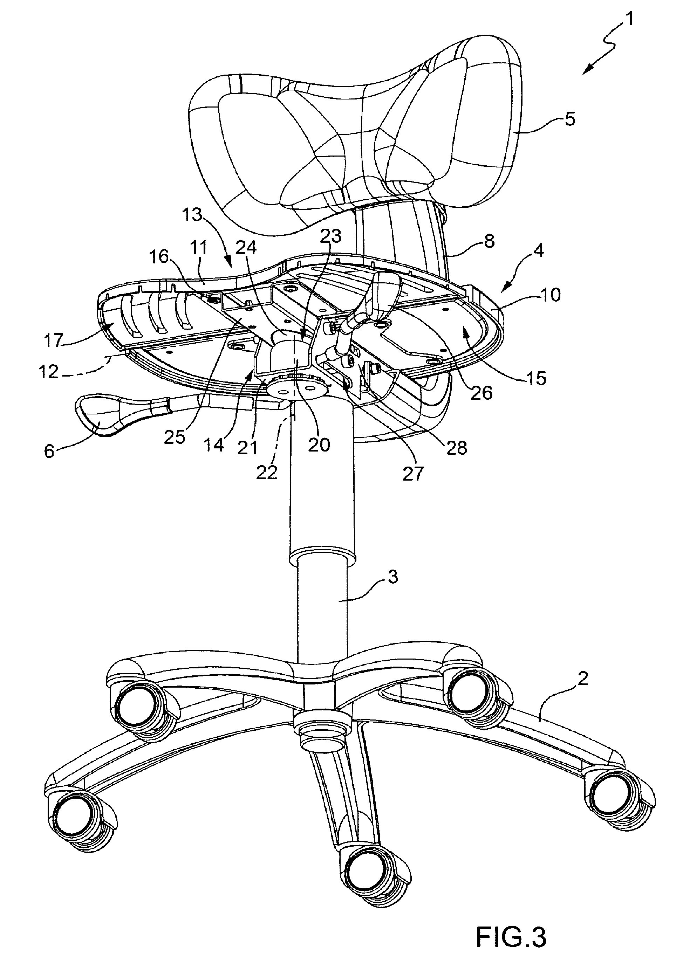

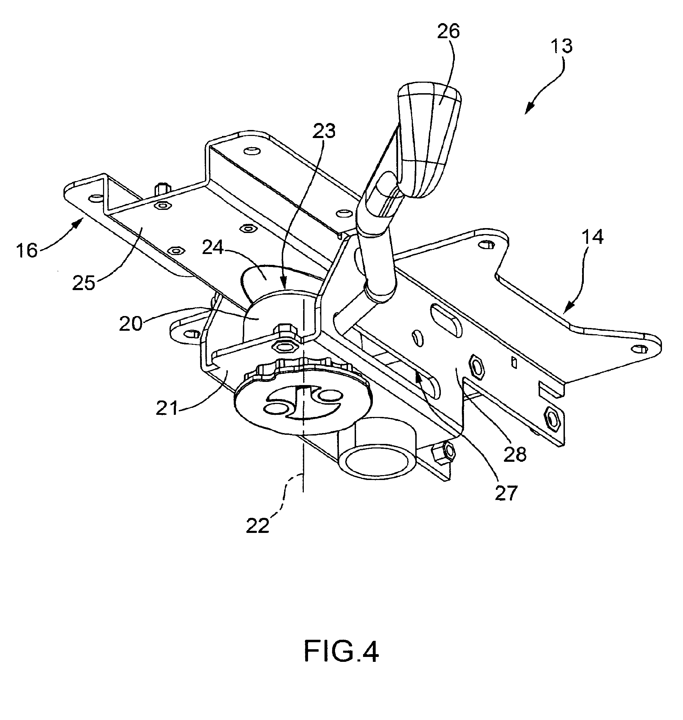

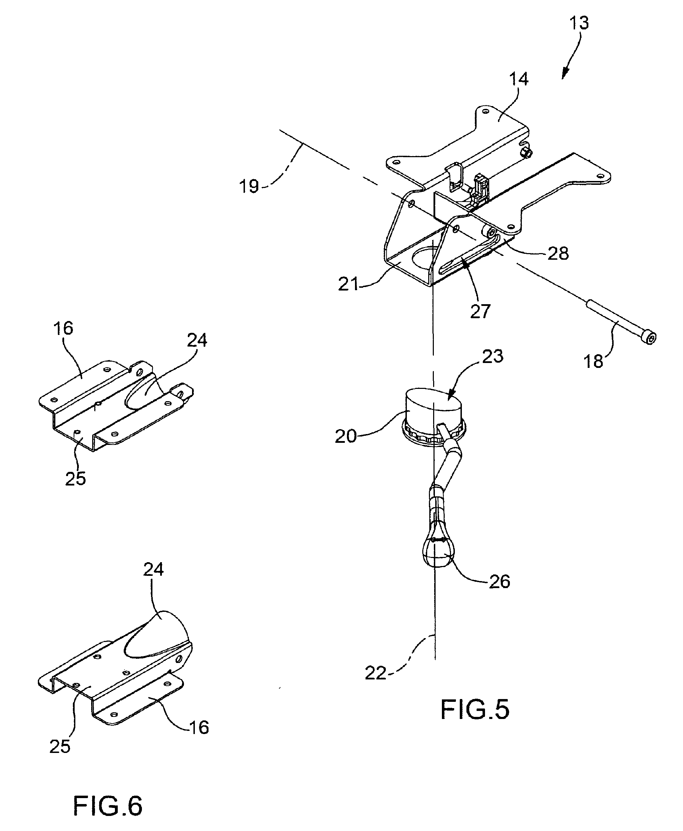

According to what is shown in Figures 3, 4, and 5, portion 11 is shifted between its lifted position and its lowered position through an operating device 13 comprising a first profile 14, which is essentially U-shaped, and is fixed to a lower face 15 of portion 10 with its concavity towards face 15 itself, and a second profile 16, which is essentially U-shaped, and is fixed to the lower face 17 of portion 11 with its concavity towards face 17 itself.

The two profiles 14 and 16 are rotatably coupled through a coupling pin 18 to rotate one relative to the other around the longitudinal axis of pin 18 parallel to axis 12.

Moreover, device 13 comprises a wedge element 20, which is substantially cylindrical, and is rotatably mounted through an end wall 21 of profile 14 to rotate, relative to profile 14 itself, around a vertical rotation axis 22, parallel to axis 7.

Wedge element 20 has a upper flat face 23, which is inclined according to an angle different from 90° relative to axis 22, and is in contact with an inclined ramp 24, obtained in an end wall 25 of profile 16, and having, in the case in point, a convex surface with a tapered shape starting from one end towards an intermediate point of profile 16 itself.

Wedge element 20 is integral with a free end of an operating lever 26, which is arranged beneath seat 4 in a substantially horizontal plane, and extends through a slit 27 obtained from a side wall 28 of profile 14.

Since the diameter of element 20 is larger than the width of ramp 24, the rotation of element 20 around axis 22, and therefore, of face 23 on ramp 24 entails the shifting of portion 11 from its lifted position to its lowered position.

In other words, when the upper edge of face 23 is in contact with ramp 24, portion 11 is in the lifted position, while when upper edge of face 23 is not in contact with ramp 24, portion 11 is in its lowered position.

Obviously, the continuous movement of lever 26 allows portion 11 to assume any position comprised between the lifted position and the lowered position.

According to a non illustrated embodiment, portion 11 can take a plurality of intermediate positions, each differing from the preceding for an angle of e.g. 2°.

The rotation of lever 26 around axis 22, i.e. around a vertical rotation axis, allows the dentist to operate lever 26 with the back of his/her hand, and prevents, therefore, that blood and/or saliva traces, that might be present on dentist's hands, contaminate lever 6 surface itself.

Relating to all said above, it should be pointed out that the inclination of face 23 and the shape of ramp 24 are such that:

wedge element 20 is time by time blocked around axis 22 by the friction existing between face 23 and ramp 24, and can be shifted around axis 22 by the rotation of lever 26 only; and

face 23 and ramp 24 are always in contact for at least a contact line.

During use, the dentist seats on stool 1, and adjusts the height of seat 4 through lever 6, the height of back rest 5, the depth of backrest 5 through lever 9, and the position of front portion 11 through lever 26, in order to select the most comfortable angle between his/her upper and lower leg.

According to some non illustrated embodiments:
ramp 24 has a flat surface; and
ramp 24 is removed and element 20 is directly in contact with portion 11.

## Claims

1. Stool, in particular for dentistry, comprising a base (2); a support post (3) protruding upwards from base (2); a seat (4) comprising, in its turn, a rear portion (10) fixed to an upper end of the supporting post (3) and a front portion (11) rotatably coupled to the rear portion (10) to rotate between a lifted position and a lowered position; and a first operating device (13) to shift the front portion (11) between said lifted and lowered positions; and
**characterized in that** said
first operating device (13) comprises a wedge element (20), which is rotatably coupled to the rear portion (10) to rotate around a first vertical rotation axis (22), has an upper face (23) inclined according to an angle different from 90° relative to the first axis (22), and supports the front portion (11) at its bottom.

2. Stool according to claim 1, wherein the first operating device (13) further comprises an inclined ramp (24), which is obtained on the front portion (11), in contact with the wedge element (20), shaped to allow the front portion (11) to shift between said lifted and lowered position following the shifting of the edge element (20) around said first axis (22) and on the inclined ramp (24) itself.

3. Stool according to claim 2, wherein the wedge element (20) has a cylindrical shape and a diameter larger than the width of the inclined ramp (24).

4. Stool according to claim 2 or 3, wherein the inclined ramp (24) has a convex surface in contact with the upper face (23) of the wedge element (20).

5. Stool according to claim 2 or 3, wherein the inclined ramp (24) has a flat surface in contact with the upper face (23) of the wedge element (20).

6. Stool according to any of the preceding claims, wherein the upper face (23) of the wedge element (20) is flat.

7. Stool according to any of the preceding claims, wherein the first operating device (13) further comprises a first operating lever (26) to rotate the wedge element (20) around the first axis (22).

8. Stool according to any of the preceding claims, wherein the front portion (11) is mounted to rotate relative to the rear portion (10) around a second horizontal rotation axis (12).

9. Stool according to any of the preceding claims and further comprising a second operating device, which selectively adjusts the height of the seat (4), and comprises a second operating lever (6) mounted to rotate around a third vertical rotation axis (7), parallel to the first axis (22).

10. Stool according to any of the preceding claims and further comprising a backrest (5) vertically and horizontally adjustable relative to the seat (4).

## Patentansprüche

1. Hocker, insbesondere für die Zahnmedizin, umfassend eine Basis (2); eine Tragsäule (3), die von der Basis (2) nach oben ragt, einen Sitz (4), der seinerseits ein rückwärtiges Teil (10) umfasst, befestigt an einem oberen Ende der Tragsäule (3) und ein Frontteil (11), das drehbar an das rückwärtige Teil (10) angeschlossen ist, um zwischen einer angehobenen und einer abgesenkten Position verdreht zu werden; und eine erste Bedienungseinrichtung (13) zum Verschieben des Frontteiles (11) zwischen der angehobenen und der abgesenkten Position; und
**dadurch gekennzeichnet, dass** die erste Bedienungseinrichtung (13) ein Keilelement (20) umfasst, das an das rückwärtige Teil (10) drehbar angeschlossen ist, um um eine erste vertikale Drehachse (22) zu drehen, und eine obere Fläche (23) aufweist, geneigt gemäß einem von 90° relativ zur ersten Achse (22) abweichenden Winkel, und dass das Frontteil (11) an seinem Boden trägt.

2. Hocker nach Anspruch 1, wobei die erste Bedienungseinrichtung (13) weiterhin eine geneigte Rampe (24) umfasst, die in Kontakt mit dem Keilelement (20) auf dem Frontteil (11) aufgenommen ist, derart gestaltet, dass sich das Frontteil (11) zwischen der angehobenen und der abgesenkten Position verschieben kann, folgend dem Verschieben des Keilelementes (20) um die genannte erste Achse (22) und auf der geneigten Rampe (24) selbst.

3. Hocker nach Anspruch 2, wobei das Keilelement (20) eine zylindrische Gestalt und einen Durchmesser aufweist, der größer als die Breite der geneigten Rampe (24) ist.

4. Hocker nach Anspruch 2 oder 3, wobei die geneigte Rampe (24) eine konvexe Fläche in Kontakt mit der oberen Fläche (23) des Keilelementes (20) aufweist.

5. Hocker nach Anspruch 2 oder 3, wobei die geneigte Rampe (24) eine erste ebene Fläche in Kontakt mit der oberen Fläche (23) des Keilelementes (20) aufweist.

6. Hocker nach einem der vorausgegangenen Ansprüche, wobei die obere Fläche (23) des Keilelementes (20) flach ist.

7. Hocker nach einem der vorausgegangenen Ansprüche, wobei die erste Bedienungseinrichtung (13) weiterhin einen ersten Bedienungshebel (26) umfasst, um das Keilelement (20) um die erste Achse (22) zu verdrehen.

8. Hocker nach einem der vorausgegangenen Ansprüche, wobei das Frontteil (11) derart montiert ist, dass es relativ zum rückwärtigen Teil (10) um eine zweite horizontale Drehachse (12) verdrehbar ist.

9. Hocker nach einem der vorausgegangenen Ansprüche und weiterhin umfassend eine zweite Bedienungseinrichtung, die selektiv die Höhe des Sitzes (4) justiert und die einen zweiten Bedienungshebel (6) umfasst, derart montiert, dass er um eine dritte vertikale Drehachse (7) verdrehbar ist, parallel zur ersten Achse (22).

10. Hocker nach einem der vorausgegangenen Ansprüche und weiterhin umfassend eine Rückenlehne (5), die vertikal und horizontal relativ zum Sitz (4) justierbar ist.

## Revendications

1. Tabouret, en particulier pour dentisterie, comprenant une base (2) ; un montant de support (3) faisant saillie vers le haut depuis la base (2) ; une assise (4) comprenant, à son tour, une portion arrière (10) fixée à une extrémité supérieure du montant de support (3) et une portion avant (11) couplée de manière rotative à la portion arrière (10) de façon à tourner entre une position levée et une position abaissée ; et un premier dispositif de commande (13) pour décaler la portion avant (11) entre lesdites positions levée et abaissée ; et
**caractérisé en ce que**
ledit premier dispositif de commande (13) comprend un élément formant coin (20), qui est couplé de manière rotative à la portion arrière (10) de façon à tourner autour d'un premier axe de rotation vertical (22), a une face supérieure (23) inclinée d'un angle différent de 90° par rapport au premier axe (22), et supporte la portion avant (11) par sa partie inférieure.

2. Tabouret selon la revendication 1, dans lequel le premier dispositif de commande (13) comprend en outre une rampe inclinée (24), qui est obtenue sur la portion avant (11), en contact avec l'élément formant coin (20), ayant une forme permettant un décalage de la portion avant (11) entre lesdites positions levée et abaissée suivant le décalage de l'élément formant coin (20) autour dudit premier axe (22) et sur la rampe inclinée (24) elle-même.

3. Tabouret selon la revendication 2, dans lequel l'élément formant coin (20) a une forme cylindrique et un diamètre plus grand que la largeur de la rampe inclinée (24).

4. Tabouret selon la revendication 2 ou 3, dans lequel la rampe inclinée (24) a une surface convexe en contact avec la face supérieure (23) de l'élément formant coin (20).

5. Tabouret selon la revendication 2 ou 3, dans lequel la rampe inclinée (24) a une surface plate en contact avec la face supérieure (23) de l'élément formant coin (20).

6. Tabouret selon l'une quelconque des revendications précédentes, dans lequel la face supérieure (23) de l'élément formant coin (20) est plate.

7. Tabouret selon l'une quelconque des revendications précédentes, dans lequel le premier dispositif de commande (13) comprend en outre un premier levier de commande (26) pour faire tourner l'élément formant coin (20) autour du premier axe (22).

8. Tabouret selon l'une quelconque des revendications précédentes, dans lequel la portion avant (11) est montée de façon à tourner par rapport à la portion arrière (10) autour d'un deuxième axe de rotation horizontal (12).

9. Tabouret selon l'une quelconque des revendications précédentes et comprenant en outre un deuxième dispositif de commande, qui règle de manière sélective la hauteur de l'assise (4), et comprend un deuxième levier de commande (6) monté de façon à tourner autour d'un troisième axe de rotation vertical (7), parallèle au premier axe (22).

10. Tabouret selon l'une quelconque des revendications précédentes et comprenant en outre un dossier (5) pouvant être réglé verticalement et horizontalement par rapport à l'assise (4).
